# FASCICULE DE BREVET EUROPEEN

(11) **EP 3 691 548 B1**
(45) Date de publication et mention de la délivrance du brevet: **06.09.2023**
(21) Numéro de dépôt: 18779725.3
(22) Date de dépôt: 08.10.2018
(51) Int. Cl.: A61B 17/06

(54) **FIL CHIRURGICAL**
CHIRURGISCHES NAHTMATERIAL
SURGICAL THREAD

(30) Priorité: 06.10.2017 EP 17195269
(43) Date de publication de la demande: 12.08.2020
(73) Titulaire: Thread & Lift, 1200 Bruxelles (BE)
(72) Inventeur: FOUMENTEZE, Jean-Paul, 06220 Vallauris (FR); FOUMENTEZE, Vincent, 1060 Saint-Gilles (BE)
(74) Mandataire: Icosa Europe
(86) Numéro de dépôt international: PCT/EP2018/077311
(87) Numéro de publication internationale: WO 2019/068928

(56) Documents cités:
- EP-A1- 3 202 336
- WO-A2-2012/116319
- US-A1- 2003 149 447
- US-B1- 8 641 732

## Description

### DOMAINE DE L'INVENTION

La présente invention concerne un fil chirurgical. En particulier, la présente invention concerne un fil chirurgical pour la médecine, la médecine esthétique et la chirurgie esthétique utilisé notamment pour la chirurgie corrective du visage aussi appelée « lifting » du visage.

### ÉTAT DE LA TECHNIQUE

Avec le temps, les tissus sous-cutanés du visage appelés aussi hypoderme ou graisse sous-cutanée s'affaissent, entraînant avec eux la peau du visage. Cet affaissement est reconnu comme étant principalement le fruit de la gravité agissant sur le visage dont les fibres conjonctives se relâchent ainsi que de la résorption périphérique osseuse. Ce vieillissement est médicalement appelé « ptose » du visage.

On connaît pour éviter l'affaissement de la peau du visage, plusieurs techniques chirurgicales comprises sous le nom générique « lifting ». Ce lifting peut être dit « malaire », « cervico-facial », ou autre selon la zone traitée ainsi que son *modus operandi.* Il consiste d'une façon générale à pratiquer une incision dans la peau, à la soulever, à repositionner les tissus sous-cutanés et musculaires superficiels puis à exciser le surplus de peau ascensionnée. Cette technique permet de traiter le problème de « ptose » mais tend à laisser des cicatrices le long des différentes incisions ainsi qu'à endommager irrémédiablement les tissus sous-cutanés. De plus, cette technique est souvent refusée par le patient en raison du résultat irréversible et pas toujours naturel.

Une pratique plus récente consiste à utiliser des fils chirurgicaux permettant de repositionner les tissus sous-cutanés affaissés du visage. Cette technique n'implique pas d'actes chirurgicaux majeurs. Ces fils sont implantés sous la peau du visage à l'aide d'aiguilles à bout mousse ne laissant aucune marque visible.

On connaît en particulier la demande de brevet US2007/0293892 décrivant un fil chirurgical pour chirurgie plastique comprenant une partie centrale et deux parties distales, les parties distales comprenant des projections pour ancrer le fil dans les tissus sous-cutanés et musculaires superficiels. Les projections de chaque partie distale sont orientées du même côté de manière à permettre le passage du fil à travers la peau dans un sens et à bloquer le glissement du fil dans le sens opposé par l'accroche des projections dans les tissus sous-cutanés et/ou musculaires superficiels du patient. Les projections sont obtenues en découpant dans le sens longitudinal du fil, une fine partie qui, en se redressant, va former une projection. Ce type de projection ainsi formée comprendra obligatoirement une extrémité distale pointue. Cette pointe à l'extrémité de la projection présente l'inconvénient d'être très agressive pour les tissus sous-cutanés et/ou musculaires superficiels du patient et est donc très douloureuse lors de la pose et dans la vie quotidienne du patient.

On connaît également la demande internationale WO2005/096956 décrivant un fil similaire dont les projections sont réalisées en découpant dans le corps de fil des parties qui vont se redresser. Ces projections permettant l'ancrage du fil dans les tissus sous-cutanés et musculaires superficiels sont arrangées en spirale autour du fil.

On connaît en outre le document WO2016/135474 décrivant un fil de suture résorbable comprenant des éléments biorésorbables de forme tronconique comprenant un alésage le long de leur axe longitudinal permettant de faire passer le fil de suture.

Cependant, ces deux dernières solutions présentent l'inconvénient de ne pas s'accrocher de manière optimale aux tissus sous-cutanés et musculaires superficiels.

On connaît enfin le document US 2003/0149447 A1 décrivant un fil chirurgical comprenant des projections. Le fil entier avec ses projections est couvert par une couche.

La présente invention vise à mettre au point un fil chirurgical permettant d'optimiser la tenue du fil dans les tissus sous-cutanés et/ou musculaires superficiels, et présentant des propriétés mécaniques adaptées et un procédé de fabrication simple et reproductible.

### RÉSUMÉ

La présente invention a pour objet un fil chirurgical défini dans la revendication 1.

La structure du fil chirurgical en deux éléments, à savoir d'une part une âme formée par le fil central et d'autre part une gaine comprenant les moyens d'accrochage sous forme de crans coniques, permet de conférer à chaque élément des propriétés mécaniques adaptées à sa fonction. En particulier, l'âme formée par le fil central, qui assure la tenue mécanique globale du fil chirurgical, doit présenter une bonne résistance à l'allongement et une bonne résistance à la rupture tout en restant souple. De préférence, le fil central présente peu ou pas d'élasticité. La gaine, qui porte les crans coniques d'accrochage, doit présenter une bonne rigidité pour éviter le retournement des crans coniques sous la pression des fibres accrochées, tout en ayant une interaction douce et flexible avec les tissus mous du patient permettant d'éviter leur inflammation et /ou leur fragilisation. La structure du fil chirurgical en deux éléments permet d'obtenir des propriétés mécaniques optimales du fil chirurgical en concevant les deux éléments indépendamment l'un de l'autre, ce qui n'est pas possible dans le cas d'un fil chirurgical monobloc où un compromis est nécessaire pour concilier les propriétés mécaniques requises pour la partie principale du fil et les moyens d'accrochage.

Selon un mode de réalisation, le fil central et la gaine sont à base d'un même matériau ayant des caractéristiques différentes pour l'un et l'autre élément, notamment en raison d'un traitement, un état (en particulier pâteux) ou une mise en oeuvre différente du matériau pour chaque élément. A titre d'exemple, le fil central et la gaine peuvent être à base d'un même matériau polymère linéaire, où le matériau polymère de la gaine a subi un procédé d'étirage que n'a pas subi le matériau polymère du fil central, de sorte que la gaine présente un module d'élasticité selon l'axe d'étirage plus élevé que celui du fil central.

Selon un autre mode de réalisation, le fil central et la gaine sont à base de matériaux différents, notamment à base de matériaux polymères différents, choisis pour conférer des propriétés mécaniques spécifiques à chaque élément. Dans un mode de réalisation, le fil central est en polyester ou en polyéthylène téréphtalate, et/ou la gaine est en silicone ou en élastomère polyuréthane.

Dans un mode de réalisation, le fil central comprend un matériau textile comportant des filaments, notamment des filaments enchevêtrés, tressés ou tissés. En particulier, les filaments peuvent être à base de matériau polymère, par exemple des filaments de polyester ou de polyéthylène téréphtalate. Une structure textile du fil central permet d'atteindre de bons niveaux de résistance à la rupture, tout en préservant la souplesse du fil central. En effet, la résistance mécanique du fil central peut être augmentée en augmentant le nombre de filaments, plutôt que par une augmentation du diamètre des filaments qui conduirait à une rigidité trop importante.

Grâce à la structure du fil chirurgical selon l'invention en deux éléments, il est également possible de munir le fil central de gaines à certains emplacements, et de le laisser dépourvu de gaine à d'autres emplacements, notamment au niveau de portions destinées à coopérer avec une aiguille ou de portions de repérage pour le chirurgien, qui n'ont pas à être munies de moyens d'accrochage.

Conformément à l'invention, les moyens d'accrochage sont conformés avec la gaine, c'est-à-dire venus de fabrication avec la gaine, directement sous forme de crans coniques présentant chacun un angle d'inclinaison compris entre 10° et 75° avec l'axe longitudinal du fil chirurgical, de manière à assurer un accrochage dans les tissus. La gaine intégrant les crans coniques est fabriquée en une seule étape, sans nécessité de reprise pour former les crans, notamment sans découpage ou autre mise en forme des crans dans une autre étape de fabrication. Il en résulte un procédé de fabrication simple du fil chirurgical. De plus, la forme du cran conformé peut avantageusement être définie pour obtenir une accroche optimisée dans les tissus.

Dans un mode de réalisation, les crans coniques sont obtenus par moulage de la gaine, notamment par moulage par injection. Selon une réalisation, le fil chirurgical est obtenu par surmoulage de la gaine autour du fil central, en plaçant le fil central dans un moule et en injectant le matériau de la gaine dans le moule autour du fil central.

La fabrication de la gaine par moulage permet d'avoir une forme des crans reproductible d'un cran à un autre et d'un fil chirurgical à un autre, ce qui n'est pas le cas pour des crans découpés, notamment en raison des mouvements du fil à la découpe et du manque de précision de la découpe pour les tailles de crans considérées. De plus, la fabrication par moulage permet d'obtenir facilement une forme conique, avec une extrémité distale arrondie des crans, contrairement au découpage qui tend à créer une pointe au sommet du cran et à générer un cran ayant des côtés non uniformes. Une extrémité distale arrondie, notamment en demi-cercle, des crans peut être formée en particulier dans le prolongement géométrique d'arêtes droites du cran. De manière avantageuse, une extrémité distale arrondie de crans est moins agressive pour les tissus du patient, sans diminution de la capacité d'accrochage du cran qui est assurée principalement par la partie angulaire à la base du cran.

Dans un mode de réalisation, le fil chirurgical comprend en outre :
- une deuxième portion s'étendant longitudinalement et comprenant un fil central recouvert d'une gaine comprenant des moyens d'accrochage comprenant chacun au moins un cran conique ;
- une troisième portion dépourvue de moyen d'accrochage et située entre la première portion et la deuxième portion.

Dans un mode de réalisation, les crans coniques sont inclinés et forment un angle par rapport à l'axe longitudinal du fil chirurgical préférentiellement de sensiblement 60°.

Dans un mode de réalisation, lesdits crans coniques comprennent une extrémité distale sphérique ou arrondie.

Dans un mode de réalisation, lesdits moyens d'accrochage comprennent N crans coniques non espacés longitudinalement et régulièrement espacés angulairement d'un angle θ₁ et dans lequel N est en entier égal ou supérieur à 2.

Dans un mode de réalisation, les moyens d'accrochage comprennent le même nombre N de crans.

Dans un mode de réalisation, les crans coniques de chaque moyen d'accrochage sont décalés en rotation d'un angle θ₃ par rapport aux crans coniques du moyen d'accrochage adjacent et dans lequel l'angle θ₃ est strictement supérieur à 0° et strictement inférieur à l'angle θ₁.

Dans un mode de réalisation, les moyens d'accrochage comprennent chacun quatre crans coniques.

Dans un mode de réalisation, l'angle θ₃ est sensiblement égal à 45°.

Dans un mode de réalisation, les crans coniques sont inclinés par rapport à l'axe longitudinal du fil chirurgical dans le sens de la troisième portion.

Dans un mode de réalisation, le fil chirurgical comprend en outre une portion de prolongement à chaque extrémité longitudinale du fil chirurgical.

L'invention concerne également un dispositif médical défini dans la revendication 15.

L'invention a également pour objet un procédé de fabrication d'un fil chirurgical défini dans la revendication 16.

Selon un mode de réalisation, la gaine est obtenue par moulage et les moyens d'accrochage sont formés lors du moulage de la gaine.

Dans un mode de réalisation, la gaine est obtenue par moulage par injection. Dans un mode de réalisation, le fil chirurgical est obtenu en plaçant le fil central dans un moule et en injectant le matériau de la gaine dans le moule autour du fil central.

### DÉFINITIONS

Dans la présente invention, les termes ci-dessous sont définis de la manière suivante :
- « **Fil** » : concerne un objet flexible s'étendant longitudinalement et dont une des trois dimensions a une longueur au moins 10 fois plus grande que les deux autres dimensions. La partie cylindrique s'étendant longitudinalement le long de ce fil est appelé corps de fil.
- « **Cran** » : concerne une projection partant du corps de fil et capable d'accrocher les tissus sous-cutanés et musculaires superficiels. Un cran comprend une base au niveau du corps de fil, et une extrémité distale, du côté opposé de la base.
- « **Extrémité distale** » : concerne ici l'une des deux extrémités longitudinales d'un cran opposée à l'extrémité proximale en contact avec le corps de fil. L'extrémité distale du cran est l'extrémité du cran destinée à accrocher les tissus périphériques au passage du fil.
- « **Sensiblement** » suivi d'une valeur numérique doit être compris comme « à plus ou moins 15%, 10%, 5%, 4%, 3%, 2% ou 1% ».

### BRÈVE DESCRIPTION DES FIGURES

Les caractéristiques et avantages de l'invention apparaîtront dans la description qui va suivre de modes de réalisation d'un fil chirurgical et d'un procédé de fabrication d'un fil chirurgical selon l'invention, donnée uniquement à titre d'exemple et faite en se référant aux dessins annexés dans lesquels :
La **Figure 1** est une vue de côté de la première portion du fil chirurgical selon un mode de réalisation de la présente invention.
Les **Figure 2A****,** et **2B** sont des vues en coupe selon l'axe A de la figure 3 de la première portion du fil chirurgical selon un mode de réalisation de la présente invention.
La **Figure 3** est une vue de coté de la première portion du fil chirurgical selon un mode de réalisation où les crans ont une forme conique et une extrémité distale arrondie.
La **Figure 4** est une vue schématisée de côté du fil chirurgical selon un mode de réalisation de la présente invention comprenant deux portions comportant des moyens d'accrochage et une portion centrale dépourvue de moyens d'accrochage.
La **Figure 5** est une vue de côté d'un fil comprenant des crans cylindriques.
La **Figure 6** est une vue de côté du fil chirurgical selon un mode de réalisation de la présente invention comprenant deux portions de prolongement à ses extrémités.

### DESCRIPTION DÉTAILLÉE

La présente invention concerne un fil chirurgical pour la médecine, chirurgie esthétique et/ou la médecine esthétique comprenant au moins une portion s'étendant longitudinalement, ladite portion comprenant des crans configurés pour s'accrocher aux tissus sous-cutanés et musculaires superficiels.

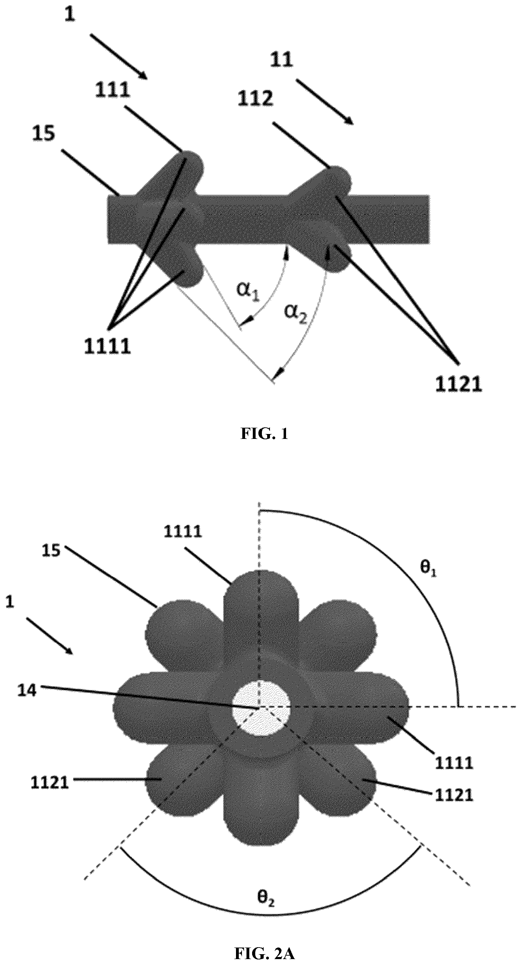

Le fil chirurgical **1** illustré sur la **Fig. 1** s'étend longitudinalement et comprend au moins une portion comprenant des moyens d'accrochage **111, 112.** Ces moyens d'accrochage **111, 112** sont arrangés longitudinalement le long de la portion du fil chirurgical 1.

Le fil chirurgical **1** comprend un fil central **14** recouvert d'une gaine **15.** Le fil central **14** peut être réalisé en polyamide (mono brin ou multi brin), polyéthylène, ou polypropylène. Préférentiellement, le fil central est en polyester ou polyéthylène téréphtalate. Selon un mode de réalisation avantageux, le fil central **14** comprend des filaments tressés, notamment des filaments de polyester ou polyéthylène téréphtalate. La gaine **15** peut être réalisée en matériau plastique, préférentiellement en matériau plastique présentant une faible variation d'allongement en fonction d'une variation de température ou de la tension subie par le fil chirurgical **1** tel que l'élastomère polyuréthane ou le silicone.

La gaine **15** comprend les moyens d'accrochage **111, 112.** Les moyens d'accrochage **111, 112** sont conformés avec la gaine **15,** sans découpage, directement sous forme de crans coniques ou sensiblement coniques présentant chacun un angle d'inclinaison compris entre 10° et 75° avec l'axe longitudinal du fil chirurgical. Dans un mode de réalisation, les moyens d'accrochage **111, 112** sont obtenus par moulage de la gaine **15.** Dans un mode de réalisation, les moyens d'accrochage **111, 112** ne sont pas obtenus par le découpage dans le sens longitudinal d'une fine partie du corps de fil. Selon un mode de réalisation, le fil chirurgical **1** est obtenu en plaçant le fil central **14** dans un moule, le matériau de la gaine **15** étant injecté dans le moule autour du fil central **14.**

Le fil chirurgical **1** comprend donc un corps de fil (ou âme) et des moyens d'accrochage **111, 112.** Le corps de fil est formé par le fil central **14** et la partie de la gaine **15** ne comprenant pas les moyens d'accrochage **111, 112.**

Dans un mode de réalisation, le corps de fil chirurgical a une épaisseur ou un diamètre qui varie de 0,25 à 0,75 mm, préférentiellement de 0,40 à 0,60 mm, et très préférentiellement de 0,45 à 0,55 mm. Dans un mode de réalisation, l'épaisseur du corps de fil est sensiblement égale à 0,25 mm, 0,30 mm, 0,35 mm, 0,40 mm, 0,45 mm, 0,50 mm, 0,55 mm, 0,60 mm, 0,65 mm, 0,70 mm ou 0,75 mm.

Comme illustré **Fig. 1****,** les moyens d'accrochage comprennent des crans **1111** et **1121** coniques ou sensiblement coniques.

Dans un mode de réalisation, le fil chirurgical **1** s'étend longitudinalement et comprend un fil central **14** et une gaine **15** ; ladite gaine **15** comprend au moins une première portion **11** et comprend des moyens d'accrochage **111, 112** comprenant chacun au moins un cran conique **1111, 1121.**

Dans un mode de réalisation, les crans n'ont pas une forme cylindrique.

Par crans coniques, on entend un volume définissant une base du cran, une extrémité distale du cran et un axe longitudinal du cran passant par le centre de la base du cran et par le centre de l'extrémité distale du cran. Enfin, la section du cran selon un axe orthogonal à l'axe longitudinal du cran est strictement décroissante depuis la base vers l'extrémité distale. Dans un mode de réalisation, les crans coniques ont plus précisément une forme tronconique.

Dans un mode de réalisation, les crans ont une hauteur qui varie de 0,30 à 1 mm, préférentiellement de 0,40 à 0,50 mm. Dans un mode de réalisation, les crans ont une hauteur sensiblement égale à 0,35 mm, 0,40 mm, 0,45 mm, 0,50 mm, 0,55 mm, 0,60 mm, 0,65 mm, 0,70 mm, 0,75 mm, 0,80 mm, 0,85 mm, 0,90 mm, 0,95 mm ou 1mm.

Par hauteur, on entend la distance entre l'extrémité distale d'un cran et le corps de fil.

Dans le mode de réalisation montré sur les figures, le cran conique a des arêtes droites. En variante, la forme du cran peut également être une combinaison entre une forme conique et une forme courbée. Dans ce cas, l'axe longitudinal du cran présente un rayon de courbure.

Dans un mode de réalisation, l'axe longitudinal du cran n'est pas confondu avec l'axe longitudinal du fil chirurgical **1** et l'axe longitudinal du cran présente un angle d'inclinaison avec l'axe longitudinal du fil chirurgical **1.**

Dans un mode de réalisation, l'angle d'inclinaison n'est pas égal à 90° ou n'est pas égal à 0°.

Dans un mode de réalisation, l'angle d'inclinaison est supérieur à 10°. Dans un mode de réalisation, l'angle d'inclinaison varie de 30 à 75°, de 35° à 70°, de 40° à 65°, de 45° à 60° ou de 50 à 55°. L'angle d'inclinaison est l'axe médian des angles α₁ et α₂ illustrés **Fig. 1****.**

Dans un plan comprenant au moins un cran et l'axe longitudinal du corps de fil comme illustré **Fig. 1****,** les deux axes à la surface du cran et opposés forment des angles α₁ et α₂ avec l'axe longitudinal du fil central **14** ou du corps de fil. Pour former des crans de forme sensiblement coniques, les angles (α₁ et α₂) formés par ces deux arêtes et l'axe longitudinal du corps de fil sont différents de manière à ce que la section du cran soit décroissante depuis la base du cran vers son extrémité distale.

Dans un mode de réalisation, l'angle α₁ est supérieur à l'angle α₂.

Dans un mode de réalisation, la différence entre ces deux angles (α₁ et α₂) varie de 30° à 5°, de 25° à 8° ou de 20° à 10°. Par « différence » entre deux angles, on entend la valeur absolue de la soustraction entre les deux angles.

Dans un mode de réalisation, α₁ varie de 80° à 30°, de 75° à 35°, de 75° à 45°, de 70° à 50°, de 65° à 55°, de 75° à 70°, de 70° à 65°, de 65° à 60°, de 60° à 55°, de 55° à 50°, ou de 62° à 58°. Dans un mode de réalisation, α₂ varie de 70° à 10°, de 65° à 15°, de 60° à 20°, de 55° à 30°, de 50° à 40°, de 30° à 35°, de 35° à 40°, de 45° à 50°, de 50° à 55°, de 55° à 60° ou de 47° à 43°.

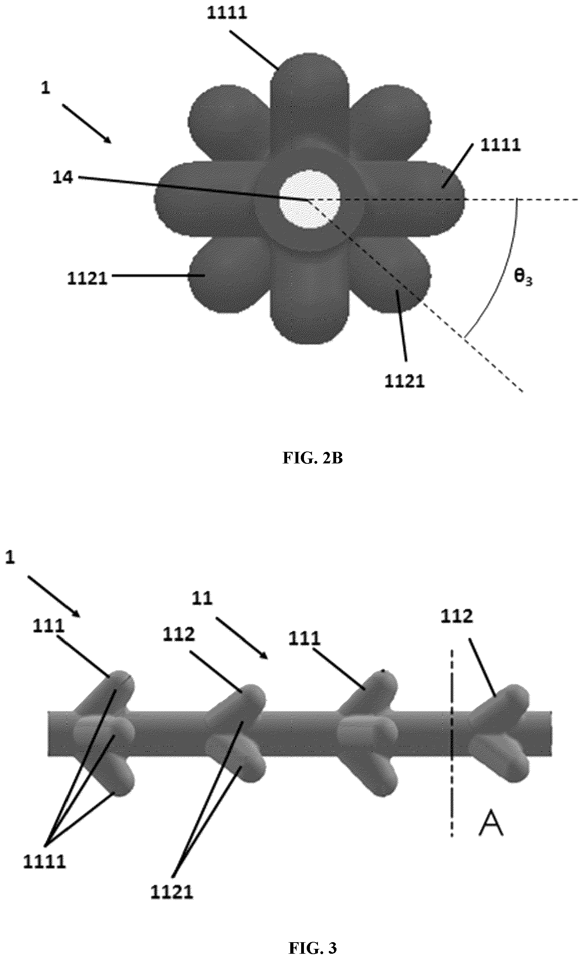

Dans un mode de réalisation illustré **Fig. 3****,** où le fil chirurgical **1** comprend une pluralité de crans sur une portion, les crans du fil chirurgical **1** de la même portion sont inclinés de manière à ce que les extrémités distales des crans « pointent » vers la même extrémité longitudinale du fil chirurgical **1.** Autrement dit, la base du cran est plus éloignée de cette extrémité du fil que l'extrémité distale du cran quand le fil chirurgical **1** est tendu.

L'angle d'inclinaison permet de renforcer l'accrochement des moyens d'accrochage dans les tissus sous-cutanés et musculaires superficiels, en minimisant le risque de déchirement et de retournement du cran en cas de mise en tension importante du dispositif. Cet effet est renforcé quand les crans sont de forme à favoriser la pénétration dans les tissus sous-cutanés et musculaires superficiels, comme décrit plus haut.

Dans un mode de réalisation, l'extrémité distale des crans des moyens d'accrochage a une forme émoussée. Préférentiellement, l'extrémité distale des crans des moyens d'accrochage a une forme sphérique ou arrondie ou sensiblement sphérique ou arrondie. Dans un mode de réalisation, l'extrémité distale des crans des moyens d'accrochage présente un rayon de courbure qui varie de 0,10 à 0,25 mm, de 0,15 à 0,21mm, de 0,10 à 0,15 mm, de 0,15 à 0,20mm, de 0,20 à 0,25mm ou préférentiellement de 0,175 à 0,185mm.

Cette forme sphérique ou arrondie de l'extrémité distale des crans facilite le retrait des fils du visage en n'entraînant ni séquelles ni marques définitives sur le visage du patient.

Dans un mode de réalisation, l'extrémité distale n'est pas située sur l'axe longitudinal du fil chirurgical **1.**

Selon l'invention, chaque moyen d'accrochage comprend au moins un cran conique.

Dans un mode de réalisation, les moyens d'accrochage comprennent N crans **1111** non espacés longitudinalement (comme illustré sur la **Fig. 3**) et régulièrement espacés angulairement d'un angle **θ₁** (comme illustré sur la **Fig. 2A**). Ainsi, les moyens d'accrochage sont constitués de N crans, situés chacun sur le même niveau longitudinal mais à différents niveaux angulaires du fil. Dans un mode de réalisation, l'angle **θ₁** (également référencé **θ₂**) est différent de 0° et de 360°. Dans un mode de réalisation, l'angle **θ₁** est égal à 360°/N. N étant un entier égal ou supérieur à 2.

Dans un mode de réalisation, les moyens d'accrochage **111** comprennent chacun le même nombre de crans **1111, 1121.** En effet, lors de l'utilisation du fil chirurgical, la tension appliquée le long du fil est mieux répartie dans le sens longitudinal lorsque les moyens d'accroche **111** et **112** comprennent tous le même nombre de crans **1111** et **1121.**

Dans un mode de réalisation les moyens d'accrochage **111** comprennent 2 crans, 3 crans, 4 crans, 5 crans, 6 crans, 7 crans, 8 crans ou au moins 9 crans.

Dans un mode de réalisation, chaque moyen d'accrochage **111** est espacé longitudinalement de son moyen d'accrochage adjacent **112** d'un pas p. Ainsi, la première portion **11** comprend un enchaînement longitudinal régulier de moyens d' accrochage **111, 112.**

Par un espacement longitudinal d'un pas p, il faut comprendre ici un espacement longitudinal d'un pas p à plus ou moins 15%, préférentiellement à plus ou moins 10%, très préférentiellement à plus ou moins 5%.

Le pas p peut varier de 0,25 mm à 10 mm. Dans un mode de réalisation préférentiel, le pas p peut varier de 1 mm à 2 mm. Très préférentiellement, le pas p peut varier de 1,25 mm à 1,75 mm. Dans un mode de réalisation, le pas p est sensiblement égal à 0,25 mm, 0,5 mm, 1 mm, 1,5 mm 2 mm, 2,5 mm, 3 mm, 3,5 mm 4 mm, 5 mm, 6 mm, 7 mm, 8 mm, 9 mm ou 10 mm.

Dans un mode de réalisation, tous les moyens d'accrochage situés sur la même portion comprennent le même nombre de crans N.

Dans un mode de réalisation illustré **Fig. 3****,** tous les moyens d'accrochage situés sur la même portion comprennent le même nombre de crans N et chaque moyen d'accrochage **111** est décalé angulairement par rapport à son moyen d'accrochage adjacent **112.**

Comme représenté sur la **Fig. 2B****,** chaque moyen d'accrochage **111** est décalé en rotation d'un angle **θ₃** par rapport à son moyen d'accrochage adjacent **112.** Dans un mode de réalisation, l'angle **θ₃** est strictement supérieur à 0° et strictement inférieur à l'angle **θ₁**. Dans un mode de réalisation, l'angle **θ₃** est différent de 0° et de 360° ou est différent d'un multiple de 360°/N. Dans un mode de réalisation l'angle **θ₃** est supérieur à 5°, 10° ou 15°. Dans un mode de réalisation préférentiel, l'angle **θ₃** est égal à 360°/(xN) où x est un entier positif égal ou supérieur à 2. Très préférentiellement, l'angle **θ₃** est sensiblement égal à 360°/(2N) ou 360°/(3N).

Dans ce mode de réalisation, l'angle de rotation entre chaque moyen d'accrochage adjacent est le même. Les contraintes sont ainsi réparties de manière sensiblement égale sur plusieurs axes réguliers. Ceci permet de répartir de la manière la plus homogène possible les contraintes appliquées par les crans du fil sur les tissus sous-cutanés et musculaires superficiels et permet d'optimiser l'efficacité de l'accrochage du fil aux tissus sous-cutanés et musculaires superficiels.

Ainsi, la portion du fil chirurgical est composée, longitudinalement, d'une pluralité de moyens d'accrochage dont les crans sont décalés angulairement avec les crans des moyens d'accrochage suivants et précédents.

Dans le mode de réalisation où l'angle **θ₃** est sensiblement égal à 360°/(2N), la portion du fil chirurgical est composée, longitudinalement, d'une pluralité de premiers moyens d'accrochage et de seconds moyens d'accrochage, et ce alternativement.

Cette alternance de premiers moyens d'accrochage et de seconds moyens d'accrochage, décalés longitudinalement et angulairement permet une meilleure accroche dudit fil chirurgical **1** dans les tissus sous-cutanés et musculaires superficiels et une meilleure tenue en traction du fil dans les tissus sous-cutanées et musculaires superficiels.

Dans un mode de réalisation préférentiel, les moyens d'accrochage **111** et **112** comprennent chacun 4 crans coniques, les angles **θ₁** sont sensiblement égaux à 90° et **θ₃** est sensiblement égal à 45°.

Dans ce mode de réalisation, la portion du fil chirurgical comprend des premiers et seconds moyens d'accrochage **111, 112** arrangés longitudinalement alternativement le long de la première portion du fil chirurgical, et les crans des premiers moyens d'accrochage **1111** sont décalés angulairement d'un angle de 45° avec les crans des seconds moyens d'accrochage **1121.**

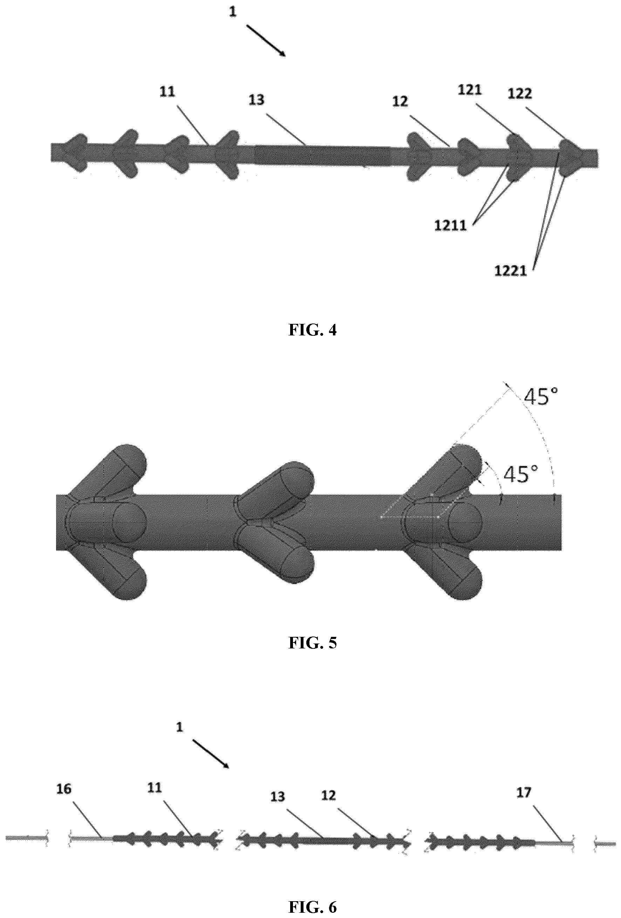

Dans un mode de réalisation illustré sur la **Fig. 4****,** le fil chirurgical **1** comprend une première portion **11** selon la présente invention et une deuxième portion **12,** similaire à la première portion **11,** ainsi qu'une troisième portion **13** dépourvue de moyens d'accrochage. La troisième portion **13** est située entre la première portion **11** et la deuxième portion **12.**

De manière similaire à la première portion **11,** la deuxième portion **12** comprend des moyens d'accrochage **121, 122** et des crans coniques **1211, 1221** dont les modes de réalisations sont les même que ceux de la première portion **11.**

Cet enchaînement de deux portions comprenant des moyens d'accrochage et une portion centrale **13** n'en comprenant pas permet d'améliorer la précision de l'accrochage des tissus sous-cutanés et musculaires superficiels. Dans ce mode de réalisation, le fil chirurgical **1** passe deux fois sous la peau, de part et d'autre de la troisième portion centrale.

Dans un mode de réalisation, au moins une portion de la troisième portion centrale **13** est colorée. Elle peut être colorée d'une couleur différente de la couleur de la première portion **11** et de la deuxième portion **12.** Dans un mode de réalisation, la troisième portion centrale **13** est colorée par une encre injectée, préférentiellement une encre de couleur noire. La coloration de cette portion centrale **13** permet au chirurgien de visualiser facilement cette portion. Dans un mode de réalisation alternatif, la troisième portion centrale **13** est colorée par l'ajout d'une gaine de silicone noir. Ainsi, le chirurgien va insérer le fil chirurgical **1** sous la peau du patient de manière à ce que l'intégralité de la première portion et la seconde portion **11, 12** soit insérée. Enfin, le chirurgien va pouvoir insérer la portion centrale **13.**

Dans un mode de réalisation, les crans des moyens d'accrochage de la première **11** et de la deuxième portion **12** sont inclinés par rapport à l'axe longitudinal du fil **1** dans le sens de la troisième portion **13.**

Par « incliné dans le sens de la troisième portion **13** », on entend ici que la base des crans au contact de l'axe longitudinal du fil chirurgical **1** est plus éloignée de la troisième portion centrale **13** que l'extrémité distale du cran correspondant.

Dans un mode de réalisation, la première portion **11** est symétrique à la deuxième portion **12** selon un axe passant par le milieu de la troisième portion centrale **13.** Dans un mode de réalisation, le fil central **14** est recouvert de deux gaines **15** selon la présente invention formant la première **11** et la deuxième portion **12.**

Dans un mode de réalisation illustré **Fig. 6****,** le fil central **14** se prolonge depuis la première portion **11** et la deuxième portion **12** dans le sens opposé à la troisième portion **13.** Ces prolongements **16, 17** sont totalement dépourvus de gaine ou de moyen d'accrochage. De cette façon, le chirurgien peut manipuler aisément les deux extrémités du fil.

Dans un mode de réalisation, ces prolongements **16, 17** du fil chirurgical **1** permettent de fixer le fil chirurgical **1** à une aiguille par sertissage ou en faisant passer l'extrémité du fil dans le chas d'une aiguille chirurgicale.

Dans ce mode de réalisation, le fil chirurgical comprend ainsi 5 portions :
- deux portions de prolongement **16** et **17** aux extrémités du fil chirurgical **1** dépourvues de moyen d'accrochage ;
- la troisième portion centrale **13** dépourvue de moyen d'accrochage ; et
- la première portion **11** et la deuxième portion **12** comprenant chacune une gaine comprenant des moyens d'accrochage et toutes deux situées entre la troisième portion centrale et une portion de prolongement.

Dans un mode de réalisation, la troisième portion centrale **13** a une longueur qui varie de 2 mm à 30 mm, 3 mm à 10 mm, de 4 mm à 6 mm, ou sensiblement égale à 5 mm. Dans un autre mode de réalisation, la troisième portion centrale **13** a une longueur qui varie de 15 mm à 25 mm, de 18 mm à 23 mm ou sensiblement égale à 20 mm.

La longueur de la première **11** et/ou de la deuxième portion **12** varie en fonction de l'endroit du visage ou du corps où le fil selon la présente invention sera utilisé. Dans un mode de réalisation, la première portion **11** et/ou la deuxième portion **12** ont une longueur qui varie de 50 à 300 mm, ou de 60 à 200 mm. Dans un mode de réalisation, la longueur de la première **11** et/ou de la deuxième portion **12** est sensiblement égale à 50, 60, 70, 80, 90, 100, 110, 120, 130, 140, 150, 160, 170, 180, 190, 200, 225, ou 250 mm.

Dans un premier mode de réalisation préférentiel, le fil chirurgical comprend une première portion **11** et une deuxième portion **12** dont la longueur varie de 80 mm à 100 mm ou de 85 mm à 95 mm et une troisième portion centrale **13** dont la longueur varie de 15 mm à 25 mm ou de 18 à 22 mm. Dans un second mode de réalisation préférentiel, le fil chirurgical **1** comprend une première portion **11** et une deuxième portion **12** dont la longueur varie de 160 mm à 135 mm ou de 150 mm à 145 mm et une troisième portion centrale **13** dont la longueur varie de 3 mm à 10 mm ou de 4 à 6 mm.

Dans un mode de réalisation, les portions de prolongement **16, 17** aux extrémités du fil chirurgical **1** dépourvues de moyens d'accrochage ont une longueur qui varie de 170 mm à 120 mm ou de 165 mm à 155 mm. Dans un autre mode de réalisation, les portions de prolongement **16, 17** aux extrémités du fil chirurgical **1** dépourvues de moyens d'accrochage ont une longueur qui varie de 160 mm à 240 mm ou de 190 mm à 210 mm. Dans un autre mode de réalisation, les portions de prolongement **16, 17** aux extrémités du fil chirurgical **1** dépourvues de moyens d'accrochage ont une longueur qui varie de 200 mm à 300 mm ou de 230 mm à 270 mm. Dans un mode de réalisation, l'épaisseur ou le diamètre des portions de prolongement **16, 17** varie de 0,10 mm à 0,35 mm, ou de 0,15 à 0,25 mm, préférentiellement de 0,20 à 0,25 mm.

Dans un mode de réalisation, le procédé de fabrication du fil chirurgical **1** comprend la formation des moyens d'accrochage lors de la fabrication de la ou chaque gaine **15** équipant le fil, directement sous forme de crans coniques présentant chacun un angle d'inclinaison compris entre 10° et 75° avec l'axe longitudinal du fil chirurgical **1.**

Dans un mode de réalisation, le fil chirurgical **1** est obtenu en plaçant le fil central **14** dans un moule et en injectant le matériau de la gaine **15** dans le moule autour du fil central.

L'invention concerne également un dispositif médical comprenant un fil chirurgical **1** selon la présente invention comprenant également une aiguille chirurgicale à une extrémité du fil chirurgical **1** ou à une portion de prolongement **16** ou **17.**

Dans un mode de réalisation, le dispositif médical comprend deux aiguilles chirurgicales, chacune étant solidarisée à une extrémité du fil chirurgical **1** ou chacune étant solidarisée à une portion de prolongement **16, 17.**

Dans un mode de réalisation, l'aiguille est solidarisée au fil chirurgical **1** par sertissage.

Dans un autre mode de réalisation, l'aiguille comprend un chas et est solidarisée au fil chirurgical **1** par le passage dudit fil **1** dans le chas de l'aiguille.

### EXEMPLES

### Matériel et Méthodes

### Matériel

On utilise deux types de fils chirurgicaux : le premier type de fil (illustré **Fig. 3**) comprend des crans de forme conique et le deuxième type de fil comprend des crans de forme cylindrique (illustré **Fig. 5**).

### Méthodes

L'objectif de ces tests est de comparer la valeur maximale de tenue à travers un tissu animal de chaque type de fil. Le fil est introduit dans un échantillon de tissu musculaire animal à l'aide d'une aiguille. L'échantillon de tissu musculaire est ensuite fixé sur la partie mobile d'un banc de traction à l'aide d'un support de maintien. L'extrémité du fil est fixée sur une pince auto-serrante de manière à tirer dans le sens opposé des crans. La vitesse du banc de traction est réglée à 30 mm/min. L'effort maximal mesuré après passage complet du fil à travers le tissu animal est relevé.

Les essais ont été réalisés sur de la noix de porc afin d'avoir un milieu de pose le plus homogène possible et afin d'éliminer les risques d'avoir la présence d'un nerf ou d'une densité musculaire différente pouvant perturber les tests comparatifs. Afin que les résultats ne soient pas dépendants de la température, les échantillons de tissus animal sont mis à température ambiante 30 min avant les tests.

### Résultats

Sur un total de 20 échantillons d'une longueur de 45 mm de chaque type de fil, la moyenne de la force maximale mesurée avec les fils comprenant des crans de forme conique est de 4,80 N tandis que celle pour les fils comprenant des crans de forme cylindrique est de 3,98 N (voir tableau ci-dessous).

| | **Fil picots coniques** | **Fil picots cylindriques** |
|---|---|---|
| | Effort mesuré (N) | |
| Échantillon 1 | 3,06 | 2,64 |
| Échantillon 2 | 4,68 | 3,86 |
| Échantillon 3 | 4,94 | 3,44 |
| Échantillon 4 | 4,28 | 2,18 |
| Échantillon 5 | 5,18 | 3,38 |
| Échantillon 6 | 3,94 | 4,02 |
| Échantillon 7 | 4,48 | 3,06 |
| Échantillon 8 | 4,10 | 4,06 |
| Échantillon 9 | 5,98 | 4,06 |
| Échantillon 10 | 3,10 | 4,28 |
| Échantillon 11 | 5,44 | 4,70 |
| Échantillon 12 | 6,04 | 4,88 |
| Échantillon 13 | 5,70 | 5,72 |
| Échantillon 14 | 4,58 | 4,16 |
| Échantillon 15 | 5,86 | 2,78 |
| Échantillon 16 | 4,72 | 3,98 |
| Échantillon 17 | 5,22 | 4,46 |
| Échantillon 18 | 5,10 | 5,46 |
| Échantillon 19 | 4,30 | 3,80 |
| Échantillon 20 | 5,28 | 4,70 |
| **MOYENNE (N)** | **4,80** | **3,98** |

La force maximale d'un fil comprenant des crans coniques est donc supérieure de plus de 20% à celle d'un fil comprenant des crans cylindriques. La forme conique des picots du fil permet donc une meilleure accroche dans les tissus qu'une forme cylindrique.

### RÉFÉRENCES

1 - Fil chirurgical ;
11 - Première portion ;
111, 112 - Moyens d'accrochage de la première portion ;
1111, 1121 - Crans coniques de la première portion ;
12 - Deuxième portion ;
121, 122 - Moyens d'accrochage de la deuxième portion ;
1211, 1221 - Crans coniques de la deuxième portion ;
13 - Troisième portion ;
14 - Fil central ;
15 - Gaine ;
16 - Première portion de prolongement ;
17 - Seconde portion de prolongement.

## Revendications

1. Fil chirurgical (1) comprenant au moins une première portion (11) s'étendant longitudinalement et comprenant un fil central (14) recouvert d'une gaine (15), ladite gaine (15) comprenant des moyens d'accrochage (111, 112) comprenant chacun au moins un cran conique (1111, 1121) dans lequel les moyens d'accrochage (111, 112) sont conformés avec la gaine (15) sans nécessité de reprise pour former les moyens d'accrochage (111, 112), sous forme de crans coniques (1111, 1121) présentant chacun un angle d'inclinaison compris entre 10° et 75° avec l'axe longitudinal du fil chirurgical (1) et **caractérisé en ce que** le fil central (14) ne comprend pas de moyens d'accrochage.

2. Fil chirurgical (1) selon la revendication **1,** comprenant en outre :
- une deuxième portion (12) s'étendant longitudinalement et comprenant un fil central (14) recouvert d'une gaine (15) comprenant des moyens d'accrochage (121, 122) comprenant chacun au moins un cran conique (1211, 1221) ;
- une troisième portion (13) dépourvue de moyen d'accrochage et située entre la première portion (11) et la deuxième portion (12).

3. Fil chirurgical (1) selon l'une quelconque des revendications **1** à **2,** dans lequel les crans coniques (1111, 1121, 1211, 1221) sont inclinés et forment un angle par rapport à l'axe longitudinal du fil chirurgical préférentiellement à sensiblement 60°.

4. Fil chirurgical (1) selon l'une quelconque des revendications **1** à **3,** dans lequel lesdits crans coniques comprennent une extrémité distale sphérique ou arrondie.

5. Fil chirurgical (1) selon l'une quelconque des revendications **1** à **4,** dans lequel lesdits moyens d'accrochage (111, 112, 121, 122) comprennent N crans coniques (1111, 1121, 1211, 1221) non espacés longitudinalement et régulièrement espacés angulairement d'un angle θ₁ et dans lequel N est en entier égal ou supérieur à 2.

6. Fil chirurgical (1) selon la revendication **5,** dans lequel les moyens d'accrochage (111, 112, 121, 122) comprennent le même nombre N de crans (1111, 1121, 1211, 1221).

7. Fil chirurgical (1) selon la revendication **6,** dans lequel les crans coniques de chaque moyen d'accrochage (111, 121) sont décalés en rotation d'un angle θ₃ par rapport aux crans coniques du moyen d'accrochage adjacent (112, 122) et dans lequel l'angle θ₃ est strictement supérieur à 0° et strictement inférieur à l'angle θ₁.

8. Fil chirurgical (1) selon la revendication **7,** dans lequel l'angle θ₃ est sensiblement égal à 45°.

9. Fil chirurgical (1) selon l'une quelconque des revendications **1** à **8,** dans lequel les moyens d'accrochage (111, 112, 121, 122) comprennent chacun quatre crans coniques (1111, 1121, 1211, 1221).

10. Fil chirurgical (1) selon la revendication **2,** dans lequel les crans coniques (1111, 1121, 1211, 1221) sont inclinés par rapport à l'axe longitudinal du fil chirurgical (1) dans le sens de la troisième portion (13).

11. Fil chirurgical (1) selon l'une quelconque des revendications **1** à **10,** dans lequel le fil central (14) comprend un matériau textile comportant des filaments, notamment des filaments tressés ou tissés.

12. Fil chirurgical (1) selon l'une quelconque des revendications **1** à **11,** dans lequel le fil central (14) est en polyester et/ou la gaine (15) est en silicone ou en élastomère polyuréthane.

13. Fil chirurgical (1) selon l'une quelconque des revendications **1** à **12,** dans lequel les crans coniques (1111, 1121, 1211, 1221) sont obtenus par moulage de la gaine (15).

14. Fil chirurgical (1) selon l'une quelconque des revendications **1** à **13,** comprenant en outre une portion de prolongement (16, 17) à chaque extrémité longitudinale du fil chirurgical (1).

15. Dispositif médical comprenant un fil chirurgical (1) selon l'une quelconque des revendications **1** à **14** et au moins une aiguille chirurgicale solidarisé au fil chirurgical (1) à une de ses extrémités longitudinales.

16. Procédé de fabrication d'un fil chirurgical (1) comprenant au moins une première portion (11) s'étendant longitudinalement et comprenant un fil central (14) recouvert d'une gaine (15), ladite gaine (15) comprenant des moyens d'accrochage (111, 112) comprenant chacun au moins un cran conique (1111, 1121), dans lequel les moyens d'accrochage (111, 112) sont formés lors de la fabrication de la gaine (15) sous forme de crans coniques (1111, 1121) sans nécessité de reprise pour former les moyens d'accrochage (111, 112), présentant chacun un angle d'inclinaison compris entre 10° et 75° avec l'axe longitudinal du fil chirurgical (1) et **caractérisé en ce que** le fil central (14) ne comporte pas de moyens d'accrochage.

## Patentansprüche

1. Chirurgischer Faden (1), der mindestens einen ersten Abschnitt (11) umfasst, welcher sich in Längsrichtung erstreckt und einen Kernfaden (14) umfasst, der mit einer Hülle (15) überzogen ist, wobei die Hülle (15) Verhakungsmittel (111, 112) umfasst, die jeweils mindestens einen konischen Widerhaken (1111, 1121) umfassen, wobei die Verhakungsmittel (111, 112) zusammen mit der Hülle (15) ohne Notwendigkeit der Wiederaufnahme, um die Verhakungsmittel (111, 112) zu bilden, in Form von konischen Widerhaken (1111, 1121) ausgebildet sind, welche jeweils einen Neigungswinkel im Bereich zwischen 10° und 75° zur Längsachse des chirurgischen Fadens (1) aufweisen, und **dadurch gekennzeichnet, dass** der Kernfaden (14) keine Verhakungsmittel umfasst.

2. Chirurgischer Faden (1) nach Anspruch **1,** weiter umfassend:
- einen zweiten Abschnitt (12), der sich in Längsrichtung erstreckt und einen Kernfaden (14) umfasst, welcher mit einer Hülle (15) überzogen ist, die Verhakungsmittel (121, 122) umfasst, die jeweils mindestens einen konischen Widerhaken (1211, 1221) umfassen;
- einen dritten Abschnitt (13), der keine Verhakungsmittel aufweist und sich zwischen dem ersten Abschnitt (11) und dem zweiten Abschnitt (12) befindet.

3. Chirurgischer Faden (1) nach einem der Ansprüche **1** bis **2,** wobei die konischen Widerhaken (1111, 1121, 1211, 1221) geneigt sind und in Bezug auf die Längsachse des chirurgischen Fadens einen Winkel von vorzugsweise im Wesentlichen 60° bilden.

4. Chirurgischer Faden (1) nach einem der Ansprüche **1** bis **3,** wobei die konischen Widerhaken ein kugelförmiges oder abgerundetes distales Ende umfassen.

5. Chirurgischer Faden (1) nach einem der Ansprüche **1** bis **4,** wobei die Verhakungsmittel (111, 112, 121, 122) N konische Widerhaken (1111, 1121, 1211, 1221) umfassen, die in Längsrichtung nicht beabstandet und winkelmäßig regelmäßig um einen Winkel θ₁ beabstandet sind, und wobei N eine ganze Zahl gleich oder größer als 2 ist.

6. Chirurgischer Faden (1) nach Anspruch **5,** wobei die Verhakungsmittel (111, 112, 121, 122) die gleiche Anzahl N an Widerhaken (1111, 1121, 1211, 1221) umfassen.

7. Chirurgischer Faden (1) nach Anspruch **6,** wobei die konischen Widerhaken jedes Verhakungsmittels (111, 121) in Drehrichtung um einen Winkel θ₃ in Bezug auf die konischen Widerhaken des benachbarten Verhakungsmittels (112, 122) versetzt sind, und wobei der Winkel θ₃ strikt größer als 0° und strikt kleiner als der Winkel θ₁ ist.

8. Chirurgischer Faden (1) nach Anspruch **7,** wobei der Winkel θ₃ im Wesentlichen gleich 45° ist.

9. Chirurgischer Faden (1) nach einem der Ansprüche **1** bis **8,** wobei die Verhakungsmittel (111, 112, 121, 122) jeweils vier konische Widerhaken (1111, 1121, 1211, 1221) umfassen.

10. Chirurgischer Faden (1) nach Anspruch **2,** wobei die konischen Widerhaken (1111, 1121, 1211, 1221) in Bezug auf die Längsachse des chirurgischen Fadens (1) in der Richtung des dritten Abschnitts (13) geneigt sind.

11. Chirurgischer Faden (1) nach einem der Ansprüche **1** bis **10,** wobei der Kernfaden (14) ein textiles Material umfasst, das Filamente, insbesondere geflochtene oder gewobene Filamente, umfasst.

12. Chirurgischer Faden (1) nach einem der Ansprüche **1** bis **11,** wobei der Kernfaden (14) aus Polyester besteht und/oder die Hülle (15) aus Silikon oder aus Polyurethan-Elastomer besteht.

13. Chirurgischer Faden (1) nach einem der Ansprüche **1** bis **12,** wobei die konischen Widerhaken (1111, 1121, 1211, 1221) durch Gießen der Hülle (15) erhalten werden.

14. Chirurgischer Faden (1) nach einem der Ansprüche **1** bis **13,** der weiter an jedem Längsende des chirurgischen Fadens (1) einen Verlängerungsabschnitt (16, 17) umfasst.

15. Medizinische Vorrichtung, die einen chirurgischen Faden (1) nach einem der Ansprüche **1** bis **14** und mindestens eine chirurgische Nadel umfasst, die an einem seiner Längsenden fest mit dem chirurgischen Faden (1) verbunden ist.

16. Verfahren zur Herstellung eines chirurgischen Fadens (1), der mindestens einen ersten Abschnitt (11) umfasst, welcher sich in Längsrichtung erstreckt und einen Kernfaden (14) umfasst, der mit einer Hülle (15) überzogen ist, wobei die Hülle (15) Verhakungsmittel (111, 112) umfasst, die jeweils mindestens einen konischen Widerhaken (1111, 1121) umfassen, wobei die Verhakungsmittel (111, 112) bei der Herstellung der Hülle (15) in Form von konischen Widerhaken (1111, 1121) gebildet werden, ohne Notwendigkeit der Wiederaufnahme, um die Verhakungsmittel (111, 112) zu bilden, welche jeweils einen Neigungswinkel im Bereich zwischen 10° und 75° zur Längsachse des chirurgischen Fadens (1) aufweisen, und **dadurch gekennzeichnet, dass** der Kernfaden (14) keine Verhakungsmittel umfasst.

## Claims

1. Surgical thread (1) comprising at least a first portion (11) extending longitudinally and comprising a central thread (14) covered with a sheath (15), said sheath (15) comprising fastening means (111, 112) each comprising at least one conical barb (1111, 1121), wherein the fastening means (111, 112) are formed with the sheath (15) without requiring reworking to form the fastening means (111, 112) in the form of conical barbs (1111, 1121) each having an angle of inclination between 10° and 75° with the longitudinal axis of the surgical thread (1), and **characterised in that** the central thread (14) is devoid of fastening means.

2. Surgical thread (1) according to claim **1,** further comprising:
- a second portion (12) extending longitudinally and comprising a central thread (14) covered with a sheath (15) comprising fastening means (121, 122) each comprising at least one conical barb (1211, 1221);
- a third portion (13) devoid of fastening means and situated between the first portion (11) and the second portion (12).

3. Surgical thread (1) according to any one of claims **1** to **2,** wherein the conical barbs (1111, 1121, 1211, 1221) are inclined and form an angle with respect to the longitudinal axis of the surgical thread preferentially of substantially 60°.

4. Surgical thread (1) according to any one of claims **1** to **3,** wherein said conical barbs comprise a spherical or rounded distal end.

5. Surgical thread (1) according to any one of claims **1** to **4,** wherein said fastening means (111, 112, 121, 122) comprise N conical barbs (1111, 1121, 1211, 1221) not spaced longitudinally and regularly spaced angularly by an angle θ₁ and wherein N is an integer equal to or greater than 2.

6. Surgical thread (1) according to claim **5,** wherein the fastening means (111, 112, 121, 122) comprise the same number N of barbs (1111, 1121, 1211, 1221).

7. Surgical thread (1) according to claim **6,** wherein the conical barbs of each fastening means (111, 121) are offset in rotation by an angle θ₃ with respect to the conical barbs of the adjacent fastening means (112, 122) and wherein the angle θ₃ is strictly greater than 0° and strictly less than the angle θ₁.

8. Surgical thread (1) according to claim **7,** wherein the angle θ₃ is substantially equal to 45°.

9. Surgical thread (1) according to any one of claims **1** to **8,** wherein the fastening means (111, 112, 121, 122) each comprise four conical barbs (1111, 1121, 1211, 1221).

10. Surgical thread (1) according to claim **2,** wherein the conical barbs (1111, 1121, 1211, 1221) are inclined with respect to the longitudinal axis of the surgical thread (1) in the direction of the third portion (13).

11. Surgical thread (1) according to any one of claims **1** to **10,** wherein the central thread (14) comprises a textile material including filament yarns, particularly braided or woven filament yarns.

12. Surgical thread (1) according to any one of claims **1** to **11,** wherein the central thread (14) is made of polyester and/or the sheath (15) is made of silicone or polyurethane elastomer.

13. Surgical thread (1) according to any one of claims **1** to **12,** wherein the conical barbs (1111, 1121, 1211, 1221) are obtained by moulding the sheath (15).

14. Surgical thread (1) according to any one of claims **1** to **13,** further comprising an extension portion (16, 17) at each longitudinal end of the surgical thread (1).

15. Medical device comprising a surgical thread (1) according to any one of claims **1** to **14** and at least one surgical needle attached to the surgical thread (1) at one of the longitudinal ends thereof.

16. Method for manufacturing a surgical thread (1) comprising at least a first portion (11) extending longitudinally and comprising a central thread (14) covered with a sheath (15), said sheath (15) comprising fastening means (111, 112) each comprising at least one conical barb (1111, 1121), wherein the fastening means (111, 112) are formed during the manufacture of the sheath (15) in the form of conical barbs (1111, 1121) without requiring reworking to form the fastening means (111, 112), each having an angle of inclination between 10° and 75° with the longitudinal axis of the surgical thread (1), and **characterised in that** the central thread (14) is devoid of fastening means.
